Europäisches Patentamt

European Patent Office

Office européen des brevets

(19)

(11) Veröffentlichungsnummer: **0 004 599**
**B1**

(12) **EUROPÄISCHE PATENTSCHRIFT**

(45) Veröffentlichungstag der Patentschrift:
14.10.81

(21) Anmeldenummer: 79100862.6

(22) Anmeldetag: 22.03.79

(51) Int. Cl.³: **A 61 M 1/03**, B 01 D 13/00,
G 01 N 21/17

(54) Verfahren und Einrichtung zum Feststellen eines Blutlecks in einem Hämodialysesystem.

(30) Priorität: 22.03.78 US 888859

(43) Veröffentlichungstag der Anmeldung:
17.10.79 Patentblatt 79/21

(45) Bekanntmachung des Hinweises auf die Patenterteilung:
14.10.81 Patentblatt 81/41

(84) Benannte Vertragsstaaten:
BE CH DE FR GB IT NL SE

(56) Entgegenhaltungen:
DE-A-1 936 120
DE-A-2 232 287
DE-A-2 509 640
DE-A-2 611 383
DE-A-2 631 686
DE-A-2 740 062
DE-U-7 037 103

(73) Patentinhaber: HOECHST AKTIENGESELLSCHAFT,
Postfach 80 03 20, D-6230 Frankfurt/Main 80 (DE)

(72) Erfinder: Dam, Naim Gulam, 50/35 231st. Street, Oakland
Gardens, New York 11364 (US)
Erfinder: Hufnagel, John Peter, Lester Avenue 861,
Mamaroneck N.Y. (US)

BUNDESDRUCKEREI BERLIN

## Verfahren und Einrichtung zum Feststellen eines Blutlecks in einem Hämodialysesystem

Gegenstand der Erfindung ist ein Verfahren sowie eine Sensoreinrichtung zum Feststellen von Blut im Dialysatstrom eines Hämodialysesystems mittels auf den Dialysatstrom gerichteten Lichtes, das periodisch unterbrochen wird.

Bei der Dialyse mittels einer künstlichen Niere wird dem Patienten von der Arterie Blut entnommen, in einem körperexternen Blutkreislauf durch einen Dialysator geleitet, wo es durch Dialyse künstlich entgiftet wird, und danach wieder zum Patienten rückgeführt. Der Dialysevorgang schließt die Molekularübertragung von Abfallstoff aus dem Blut zu einer Dialysatlösung durch mechanische Diffusion durch ein kolloidales semipermeables Dialysatorglied in sich ein. Die Dialysatlösung wird in einem unabhängigen Dialysatkreislauf, der vom körperexternen Blutkreislauf innerhalb des Dialysators durch das semipermeable Dialysatorglied getrennt ist, geführt. Die beiden Flüssigkeitsströme fließen zueinander parallel durch den Dialysator. Das Dialysatorglied ist von herkömmlicher Art, wie z. B. eine Massenübertragungsmembran aus Elastomermaterial von einigen Millimetern Dicke oder ein Hohlfaserbündel. Die Abfallstoffe werden durch das Dialysatorglied vorzugsweise unter Zuhilfenahme eines negativen Druckgefälles übertragen. Die Poren des Dialysatorgliedes sind zu klein, um Blut durchzulassen, jedoch genügend groß für den Durchtritt von Abfallstoffen. Bildet sich ein Leck im Dialysatorglied, wandert das Blutplasma in den Dialysatorkreislauf, was eine ernste Gefahr für den Patienten bedeutet, insbesondere dann, wenn das Dialysesystem in der bevorzugten Weise mit einer Druckdifferenz quer über das Dialysatorglied arbeitet. In letzterem Fall werden große Mengen von Blut durch das Dialysatorglied in die Dialysatorlösung gezogen. Aufgrund der für die Dialyse benötigten langen Zeitspanne (im allgemeinen etwa sechs Stunden) und des Unvermögens des Patienten sich wirksam gegen eine solche Gefahr zu schützen, ist eine automatische Lecküberwachung für ein Hämodialysesystem wichtig.

Nach der deutschen Offenlegungsschrift 2 631 686 ist ein System zum Feststellen einer Blutleckage der eingangs genannten Art bekannt. Es basiert auf der Detektion von Farbänderungen in der Dialysatlösung. Dieses System fühlt Unterschiede in der Lichtübertragung durch die Dialysatlösung als Resultat der Änderung der Absorptionseigenschaften zwischen den Bestandteilen der Dialysatlösung und dem Blutplasma ab. Um in zufriedenstellender Weise zwischen Änderungen der Absorptionseigenschaften der Dialysatlösung und dem Blut mit einem angemessenen Empfindlichkeitsgrad zu unterscheiden und um Fehlmessungen wegen eventuell vorhandener Gasblasen zu minimieren, sind zwei Lichtquellen unterschiedlicher Wellenlänge erforderlich. Das Überwachungssystem ist verhältnismäßig komplex und teuer.

Hier will die Erfindung Abhilfe schaffen.

Gemäß der Erfindung wurde entdeckt, daß bei genauer photoelektrischer Ausrichtung eines Photosenders und Photodetektors und intermittierender Aussendung eines Lichtstrahls in die Dialysatlösung sowie Messung von Änderungen in der reflektierten Lichtintensität ein hochselektives Ansprechen auf eine Blutleckage durch das Dialysatorglied möglich ist. Das erfindungsgemäße Verfahren ist gekennzeichnet durch

— Aussendung eines engen Lichtstrahls in den Dialysatstrom von einer Seite desselben in einer auf die zentrale Achse des Stroms auftreffenden Richtung unter einem vorbestimmten Einfallwinkel in bezug auf eine zur zentralen Achse normale Achse,

— Empfangen von reflektiertem Licht in einer im wesentlichen gemeinsamen Ebene mit dem engen Lichtstrahl und auf derselben Seite des Dialysatstromes, derart, daß es mit der normalen Achse einen Reflektionswinkel einschließt, der im wesentlichen gleich ist dem Einfallwinkel,

— Feststellen des durchschnittlichen Gleichstromwertes des vom reflektierten Licht erzeugten elektrischen Signals und

— Erzeugen eines Alarmsignals, sobald der durchschnittliche Gleichstromwert einen vorbestimmten Wert überschreitet.

Ein weiteres erfindungsgemäßes Merkmal liegt in der Verwendung einer »Floating reference«, die die Notwendigkeit, das System vor dem Dialysieren wieder zu kalibrieren, umgeht. Bis dato war eine Wiederkalibrierung im Hinblick auf Ansprechänderung aufgrund thermischer Abweichungen und physikalischer Änderungen der Eigenschaften der Bestandteile im Dialysat notwendig. Die »Floating reference« wird durch einen Mikroprozessor erstellt, der dieses Signal mit dem abgefühlten optischen Signal vergleicht, um zu entscheiden, ob ein Alarmsignal abgegeben werden soll. Aufgabe der Erfindung ist somit auch eine Einrichtung zum Feststellen eines Blutlecks in dem Dialysatstrom eines Hämodialysesystems anzugeben, die verläßlich, kompakt und billig ist.

Eine weitere Aufgabe der Erfindung ist, ein Verfahren und eine Einrichtung anzugeben, das bzw. die das Vorhandensein von Blut in einem Dialysatstrom mit einem Minimum an Kalibrierung feststellt.

Weitere Aufgaben und Vorteile der Erfindung ergeben sich aus der nachstehenden detaillierten Beschreibung in Verbindung mit den Zeichnungen. In letzteren zeigt

Fig. 1 ein schematisches Blockschaltbild der erfindungsgemäßen Sensoreinrichtung,

Fig. 2 einen Querschnitt durch einen bevorzugten optischen Wandler für die Einrichtung nach Fig. 1,

Fig. 3 ein Diagramm, das ein typisches Verhältnis zwischen Stromübertragung und Temperatur für einen herkömmlichen »solid-state«-Lichtabgabewandler zeigt, und

Fig. 4 ein detailliertes Schaltbild der Einrichtung nach Fig. 1.

Die Detektoreinrichtung nach der Erfindung kann mit jedem herkömmlichen Hämodialysesystem verwendet werden.

Der Blutleckdetektor wird zweckmäßig in der Dialysatverteileranordnung montiert, um das Dialysat nach dem Durchgang durch den Dialysator und vor dem Rückleiten zum Chargenbehälter bzw. Abführen als Abfall zu überwachen.

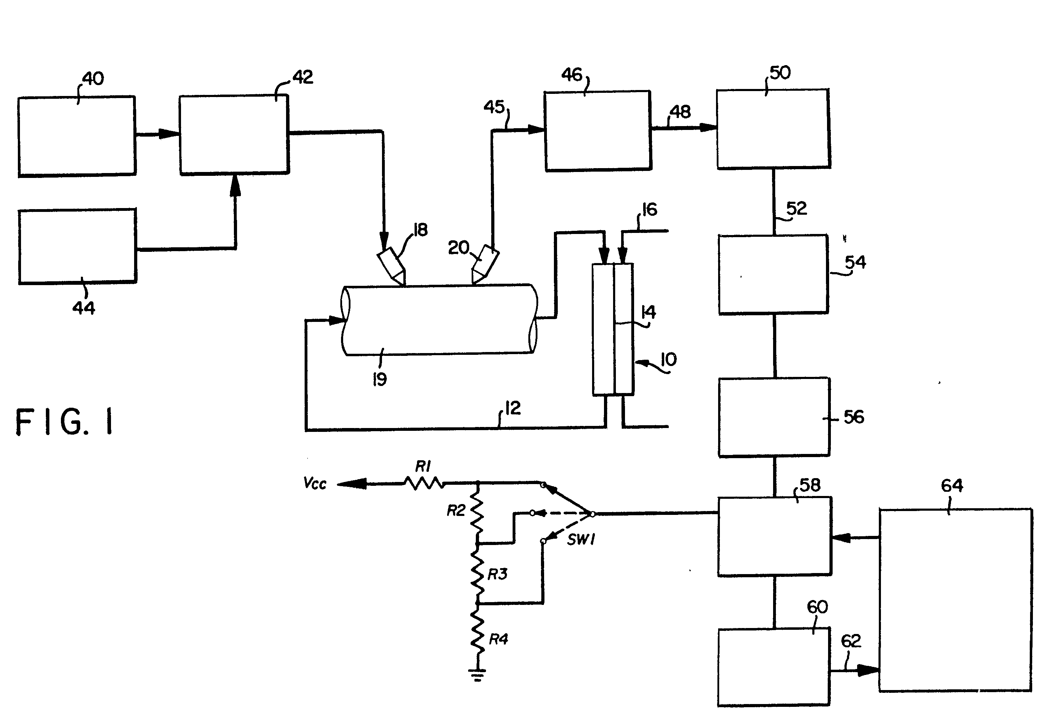

Fig. 1 zeigt diagrammatisch einen Dialysator 10 beliebiger herkömmlicher Geometrie und Konstruktion mit zwei parallelen durch ein Dialysatglied 14 getrennten Wegen. Eine Dialysatlösung zirkuliert in einem Dialysatorstromweg 12, der auf einer Seite des Dialysatorgliedes 14 durch den Dialysator 10 führt. Blutplasma fließt durch einen körperexternen Blutkreislauf 16, der auf der entgegengesetzten Seite des Dialysatorgliedes 14 durch den Dialysator führt.

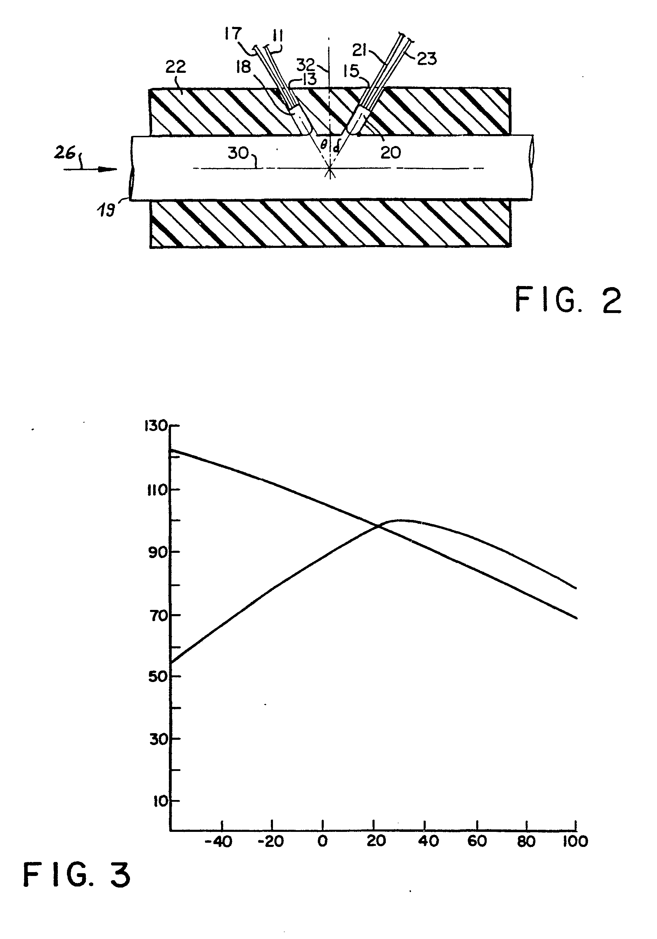

Ein optischer Sender 18 und ein optischer Empfänger 20 sind angeordnet, um Licht durch einen Abschnitt der Leitung 19 des Dialysatkreislaufes 12, der vorzugsweise stromab des Dialysators 10 angeordnet ist, zu senden. Reflektiertes Licht durch das Dialysat wird verwendet, um das Vorhandensein von Blut in der Dialysatlösung festzustellen. Der optische Sender 18 und der optische Empfänger 20 sind, wie deutlicher in Fig. 2 gezeigt ist, in einem Block 22, vorzugsweise aus Kunststoff, wie Akrylonitril-Butadienstyrol, montiert, der eine zylindrische Bohrung zur Montage des Blockes 22 um einen Abschnitt der Leitung 19 des Dialysatstromweges 12 besitzt. Der optische Sender 18 und der optische Empfänger 20 sind in Bohrungen 13 und 15 des Blockes 22 eingesetzt, um eine vorbestimmte Orientierung zwischen den Wandlerelementen 18 und 20 und dem Dialysatstrom 26 festzulegen. Elektrische Leitungen 17, 11, 21 und 23 erstrecken sich von dem Block 22 weg, was erlaubt, die Lichtwandlerelemente 18 und 20 innerhalb der Bohrungen 13 und 15, z. B. mittels eines herkömmlichen Epoxyharzes zu befestigen.

Der optische Sender 18 und der optische Empfänger 20 sind vorzugsweise in derselben Ebene auf einer gemeinsamen Seite in bezug auf den Dialysatstrom 26 angeordnet. Der Sender 18 ist auf die zentrale Achse 30 des Dialysatstromes 26 ausgerichtet, um einen vorbestimmten Einfallswinkel Θ in bezug auf die normale Achse 32 zu bilden. Der Empfänger 20 sollte mit der normalen Achse 32 einen Reflexionswinkel einschließen, der im wesentlichen gleich ist dem Einfallswinkel. Der optische Sender 18 und der optische Empfänger 20 stellen vorzugsweise eine herkömmliche Kombination eines »solid-state«-Lichtabgabediodensenders und eines Phototransistordetektors dar. Für eine Lichtstrahlabweichung von weniger als etwa 12° beträgt der bevorzugte Einfallswinkel Θ etwa 55°.

Der optische Sender 18 wird durch eine Quelle 40 konstanten Stromes über eine Treiberschaltung 42, die durch einen Oszillator mit einer vorbestimmten Frequenz intermittierend unterbrochen wird, betrieben. Das vom optischen Empfänger 20 in Form eines Photodetektors empfangene Licht wird als ein elektrisches Signal an einen Spitzenwert-Hüllkurvendetektorkreis 46 geführt, der seinerseits ein den Durchschnitts-Gleichstromwert des Eingangssignals 45 repräsentierendes Ausgangssignal 48 liefert. Ein Substraktionskreis 50 liefert, wie sein Name verrät, ein Ausgangssignal 52, das die Differenz zwischen einer festen Gleichstrom-Bezugsspannung und dem Gleichstromausgangssignal 48 darstellt. Die feste Gleichstrom-Bezugsspannung dient dazu, das System, wie nachstehend näher beschrieben, anfänglich zu kalibrieren. Das Ausgangssignal 52 wird durch den Verstärker 54 verstärkt und über eine Trennschaltung 56 einem Multiplexer 58 zugeführt, der das Signal 52 in einem Analog-Digital-Konverter 60 zur Umwandlung in ein digitalisiertes 8-Bit-Datensignal 62 überträgt. Das Datensignal wird der zentralen Verarbeitungseinheit CPU 64 eines herkömmlichen Mikroprozessors zugeführt, der programmiert ist, das Datensignal 62 zu lesen und zu speichern und es in vorbestimmten Zeitintervallen auszuwerten, um auf Basis der Einstellung des manuell zu betätigenden Empfindlichkeitsregelschalters SW1 festzustellen, ob ein Alarm ausgesendet werden soll oder nicht. Der Mikroprozessor 64 ist auch dazu programmiert, das Signal 62 für ein vorbestimmtes Zeitintervall zu tracken und das getrackte Signal als Bezugssignal zu verwenden, um darauf die Entscheidung betreffend der Auslösung eines Alarms zu basieren. Obwohl ein herkömmlicher Mikroprozessor verwendet wird, der vorzugsweise durch ein einfaches »software«-Programm gesteuert wird, versteht es sich, daß die Operation auch von einer funktionell äquivalenten Schaltung, wie einer »Sample and Hold-Schaltung« in Kombination mit einem einstellbaren Timing-Kreis, durchgeführt werden könnte.

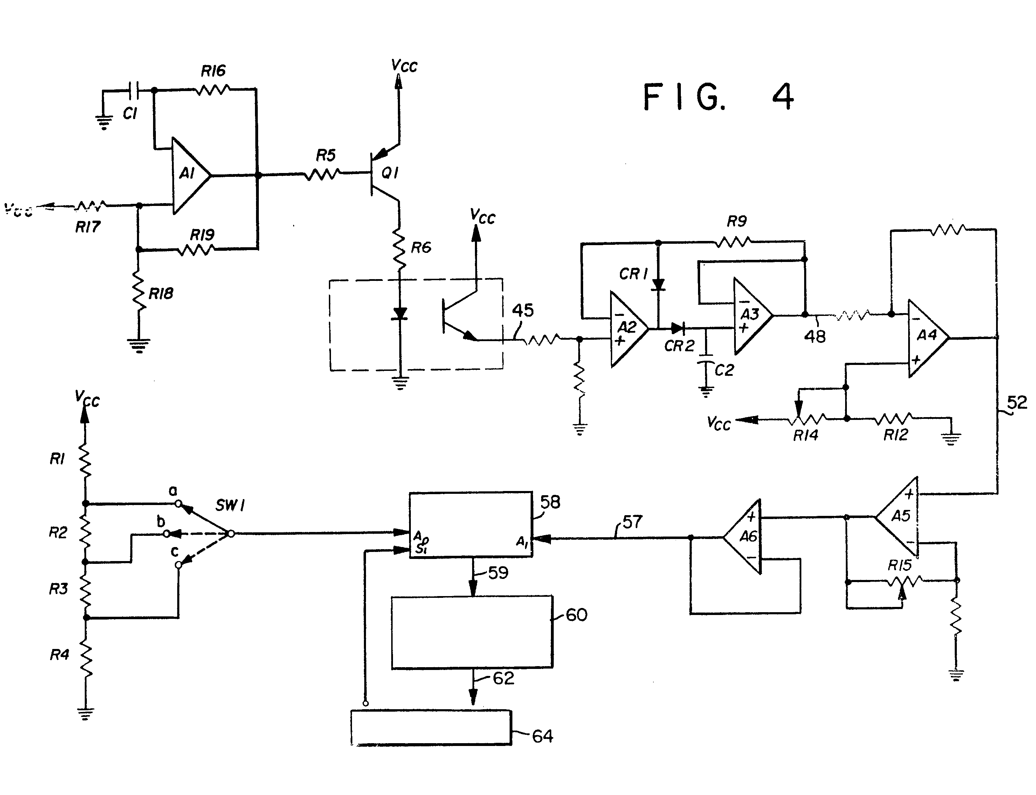

Ein detaillierteres Schaltbild der Einrichtung nach Fig. 1 ist in Fig. 4 gezeigt. Ein Operationsverstärker A1 wird als astabiler Multivibrator verwendet und stellt den Taktoszillator 44 der Fig. 1 dar. Die Schwingungsfrequenz wird durch die Werte der Widerstände R16, R17, R18, R19 und des Kondensators C1 bestimmt. Eine Schwingungsfrequenz von weniger als etwa 100 Hz wird bevorzugt. Die Quelle 40 für konstanten Strom und die Treiberschaltung 42 für den optischen Sender werden von dem Transistor Q1 in Kombination mit Widerständen R5 und R6 gebildet. Es ist wichtig, daß der Transistor Q1 mit einem Verhältnis von Einschaltzeit zu Ausschaltzeit von etwa 1 betrieben wird.

Die Bedeutung dieses Arbeitsspiels ist aus dem typischen Schaubild des Verhältnisses von Stromübertragungsverhältnis zu Umgebungstemperatur für eine lichtimittierende Diode (siehe Fig. 3) ersichtlich. Der Verlauf der Kurve ist steil, was anzeigt, daß bei jeder kleinen Änderung der Umgebungstemperatur eine relativ starke Änderung im Stromübertragungsverhältnis stattfindet, was, wenn zugelassen, wesentliche Änderungen in der Stärke des Lichtausgangssignals verursachen würde. Es wurde gefunden, daß ein intermittierender Betrieb des optischen Senders 18 mit einem Verhältnis von Einschaltzeit zu Ausschaltzeit von im wesentlichen 1 Änderungen in der Umgebungstemperatur auf ein Minimum herabsetzt.

Der Ausgang 45 des optischen Empfängers 20 ist an den Spitzenwert-Hüllkurvendetektor 46 der Fig. 1 geführt, welcher Operationsverstärker A2, A3 in Kombination mit Dioden CR1 und CR2 und einen Ladekondensator C2 umfaßt. Der Kondensator C2 lädt auf einen Gleichstromwert auf, der in Beziehung steht mit dem durchschnittlichen Gleichstromwert des Signals 45. Die Diode CR2 verhindert eine Entladung des Kondensators C2 über den Operationsverstärker A2. Die Rückführschleife des Widerstandes R9 vom Ausgang des Operationsverstärkers A3 zum Eingang des Operationsverstärkers A2 hält eine Abweichung (drift) aufgrund der Gleichstromeingangsvorspannung des Operationsverstärkers A3 auf einem Minimum. Die Diode CR1 kompensiert einen Leckstrom durch die Diode CR2.

Der Ausgang 48 der Spitzenwert-Detektorschaltung 46 ist an den Subtraktionskreis 50 von Fig. 1 geführt, welcher gemäß Fig. 4 einen Operationsverstärker A4 und eine Verschiebungsspannungs-Einstellschaltung mit einem Widerstand R12 und einem einstellbaren Widerstand R14 enthält. Vcc ist die Gleichstrom-Vorspannung der Speisequelle des Systems. Das Nichtvorhandensein von Blut in der den optischen Wandlerblock 22 passierenden Dialysatlösung erzeugt ein Lichtenergie-Schwellensignal, das als Hintergrund-Gleichstromsignal an den Ausgang 48 des Spitzenwert-Detektors 46 reflektiert wird. Das System wird anfänglich durch Ausnullung des Hintergrund-Gleichstromsignals in der Subtraktionsschaltung 52 unter Verwendung einer äquivalenten Verschiebungsspannung kalibriert. Diese anfängliche Kalibrierung wird durch Einstellung des einstellbaren Widerstandes R14, bis das Ausgangssignal 52 Null ist, durchgeführt. Das Ausgangssignal 52 wird dem Verstärker A5 zugeführt, der als Nicht-Inverter mit mittels eines einstellbaren Widerstandes R15 einstellbarer Verstärkung geschaltet ist. Der Verstärker A5 ist durch einen nicht-invertierend geschalteten Operationsverstärker A6 isoliert. Der Operationsverstärker A6 stellt die Trennschaltung 56 von Fig. 1 dar. Der Ausgang 57 des Operationsverstärkers A6 ist an den Eingang A1 des Multiplexers 58 geführt. Der Multiplexer 58 ist eine herkömmliche im Handel erhältliche Einrichtung in Form eines elektronischen Einpol-Mehrfachpositions-Schalters, dessen Positionen durch einen logischen Wert am Eingang S1 bestimmt werden. Der Eingang A0 des Multiplexers ist mit einem von Hand einstellbaren Schalter SW1 verbunden, der mehrere Schaltpositionen a, b, c besitzt, die unterschiedliche Empfindlichkeitsstufen darstellen, welche durch die ausgewählten Kombinationen von Widerständen R1, R2, R3 bzw. R4 bestimmt werden. Der Ausgang 59 des Multiplexers ist mit einem herkömmlichen Analog-Digital-Konverter, z. B. dem Modell AD 571 der Firma Analog Device Inc., verbunden. Der Analog-Digital-Konverter erzeugt ein dem Analogsignal 59 entsprechendes 8-Bit-Digitalsignal 62. Das digitalisierte 8-Bit-Datensignal 62 wird einem herkömmlichen Mikroprozessor 64 zugeführt.

Im Normalbetrieb trackt der Mikroprozessor das Eingangssignal 62, nachdem der Dialysevorgang gestartet ist, und speichert am Ende einer vorbestimmten Zeitspanne das Eingangssignal 62 als ein Bezugs-Kalibriersignal in einen Speicher. Gelangt Blut durch das Dialysatorglied 14 in den Dialysatkreislauf 12, so bewirkt das von roten Blutkörperchen, die die optische Wandleranordnung passieren, refektierte Licht eine Änderung des Gleichstrom-Ausgangssignals 48, was eine entsprechende Änderung in den zum Mikroprozessor 64 gelangenden Digitaldaten 62, verursacht, und der Mikroprozessor bestimmt seinerseits, entsprechend der vom Patienten gewählten Empfindlichkeit, ob die Änderung des Ausgangssignals genügt, um die Auslösung eines Alarms zu rechtfertigen.

**Patentansprüche**

1. Verfahren zum Feststellen des Vorhandenseins von Blut im Dialysatstrom eines Hämodialysesystems mittels auf den Dialysatstrom gerichteten Lichtes, das periodisch unterbrochen wird, gekennzeichnet durch

— Aussenden eines engen Lichtstrahls in den Dialysatstrom von einer Seite desselben in einer auf die zentrale Achse des Stroms auftreffenden Richtung unter einem vorbestimmten Einfallwinkel in bezug auf eine zur zentralen Achse normale Achse,
— Empfangen von reflektiertem Licht in einer im wesentlichen gemeinsamen Ebene mit dem engen Lichtstrahl und auf derselben Seite des Dialysatstroms, derart, daß es mit der normalen Achse einen Reflektionswinkel einschließt, der im wesentlichen gleich ist dem Einfallwinkel,
— Feststellen des durchschnittlichen Gleichstromwertes des vom reflektierten Licht erzeugten elektrischen Signals und
— Erzeugen eines Alarmsignals, sobald der durchschnittliche Gleichstromwert einen vorbestimmten Wert überschreitet.

2. Verfahren nach Anspruch 1, gekennzeichnet durch

— Abziehen einer vorbestimmten Verschiebungsspannung vom durchschnittlichen Gleichstromwert,
— Umwandeln des reduzierten Signals in ein Digitalsignal,
— Speichern des Digitalsignals als digitales Bezugssignal am Ende einer vorbestimmten ersten Zeitspanne,
— wiederholtes Vergleichen des digitalen Bezugssignals mit dem der ersten Zeitspanne folgenden Digitalsignal, und
— Erzeugen des Alarmsignals, sobald das Digitalsignal gegenüber dem digitalen Bezugssignal variiert.

3. Sensoreinrichtung zum Feststellen des Vorhandenseins von Blut im Dialysestrom eines Hämodialysesystems mittels auf den Dialysatstrom gerichteten Lichtes, das periodisch unterbrochen wird, nach Anspruch 1, gekennzeichnet durch

— eine Leitung (19) für den Dialysatstrom, einen optischen Sender (18), der auf einer Seite der Leitung (19) angeordnet ist, um, einen engen Lichtstrahl mit einem vorbestimmten Einfallswinkel (θ) in bezug auf eine zur zentralen Achse (30) der Leitung (19) normale Achse (32) in die Dialysatlösung zu senden,
— einen optischen Empfänger (20), der auf der gleichen Seite der Leitung (19) wie der Sender (18) und in im wesentlichen der gleichen Ebene unter einem vorbestimmten Reflexionswinkel (σ) in bezug auf die normale Ebene zum Empfangen reflektierten Lichtes aus der Dialysatlösung angeordnet ist, wobei der Reflexionswinkel im wesentlichen gleich ist dem vorbestimmten Einfallswinkel,
— eine Einrichtung zum Abfühlen des Ausganges des optischen Empfängers,
— eine Einrichtung zur Erzeugung eines Gleichstrom-Ausgangssignals, das auf den durchschnittlichen Gleichstromwert des abgefühlten Ausgangssignals anspricht, und
— eine auf das Gleichstrom-Ausgangssignal ansprechende Einrichtung zur Abgabe eines Alarmsignals, wenn das Gleichstrom-Ausgangssignal einen vorbestimmten Wert übersteigt.

4. Einrichtung nach Anspruch 3, dadurch gekennzeichnet, daß der vorbestimmte Einfallswinkel (θ) im wesentlichen etwa 55° beträgt.
5. Einrichtung nach Anspruch 3, dadurch gekennzeichnet, daß die Einrichtung (42) zum intermittierenden Leiten von Gleichstromimpulsen durch den optischen Sender einen Oszillator (44) sowie eine Einrichtung (40) zur Erzeugung eines konstanten Stromes während eines vorbestimmten Abschnittes jedes Oszillationszyklus umfaßt.
6. Einrichtung nach Anspruch 3, dadurch gekennzeichnet, daß die Einrichtung zur Erzeugung eines Gleichstrom-Ausgangssignals einen Spitzenwert-Hüllkurvendetektor (46) umfaßt.

7. Einrichtung nach Anspruch 3, dadurch gekennzeichnet, daß die Einrichtung zur Abgabe eines Alarmsignals einen Differenzverstärker (A4), dessen einer Eingang mit dem Gleichstromsignalausgang (48) und dessen zweiter Eingang mit dem Ausgang einer einstellbaren Gleichstrom-Bezugsquelle verbunden ist, eine Einrichtung (60) zur Umwandlung des Ausganges (52) des Differenzverstärkers (A4) in ein digitales Kontrollsignal und eine Mikrocomputereinrichtung (64) umfaßt, um das digitale Kontrollsignal für eine erste vorbestimmte Zeitspanne zu tracken, das digitale Kontrollsignal am Ende der ersten Zeitspanne als digitales Bezugssignal zu speichern und das digitale Bezugssignal mit dem digitalen Kontrollsignal, das der ersten vorbestimmten Zeitspanne folgt, zu vergleichen.

**Claims**

1. A method for detecting the presence of blood in the dialysate flowstream of a hemodialysis system by means of light directed on the dialysate flowstream and interrupted periodically comprising the steps of:

transmitting a narrow beam of light into said dialysate flowstream from one side thereof in a direction incident upon the central axis of the flowstream with a predetermined angel of incidence relative to an axis normal to said central axis;
receiving light reflected by the dialysate in a substantially common plane with said narrow beam of light and on the same side of said flowstream such that it forms an included angle of reflection with said normal axis substantially equal to said angle of incidence;
interrupting said transmission of light at a relatively low repetition rate with the ratio of on time to off time being substantially equal;
detecting the average DC level of the signal generated by said reflected light in an optical receiver and generating an alarm signal when said average DC level exceeds a predetermined level.

2. A method as defined in claim 1 further comprising the steps of:

substracting a predetermined DC offset voltage from said average DC level;
converting the substracted signal into a digital signal; storing said digital signal as a digital reference signal at the end of a first predetermined time;
repeatedly comparing said digital reference signal to said digital signal following said first predetermined time;
and generating said alarm signal when said digital signal varies from digital reference.

3. A sensor system for detecting the presence of blood in the dialysate flowstream of a hemodialysis system by means of light directed on the dialysate flowstream and interrupted periodically as defined in claim 1 comprising:

a conduit (19) through which the dialysate flows; an optical transmitter (18) arranged on one side of said conduit (19) for transmitting a narrow beam of light into said dialysate flowstream at a predetermined angle (Θ) of incidence relative to an axis (30) normal to said central axis (30) of conduit (19); an optical receiver (20) connected to said conduit (19) on the same side thereof as said transmitter (18) in substantially the same plane and at a predetermined angle (σ) of relection relative to said normal axis for receiving reflected light from said dialysate flowstream said angle of reflection being substantially equal to said predetermined angle of incidence; means for detecting tho output of said optical receiver; means for generating an output DC signal responsive to the average DC level of said detected output; and means responsive to said output DC signal for providing an output alarm signal when said output DC signal exceeds a predetermined level.

4. A system as defined in claim 3 wherein said predetermined angle (Θ) of incidence is substantially about 55°.

5. A system as defined in claim 3 wherein said means (42) for intermittently passing pulses of direct current through said optical transmitter comprises an oscillator (44) and means (40) for generating a constant current during a predetermined portion of each oscillating cycle.

6. A system as defined in claim 3 wherein said means for generating a DC output signal comprises a peak envelop detector (46).

7. A system as defined in claim 3 wherein said means for providing an output alarm signal comprises: a difference amplifier (A4) having one input connected to said output DC signal (48) and a second input connected to the output of an adjustable DC reference source, means (60) for converting the output (52) of said difference amplifier (A4) into a digital control signal; and microcomputer means (64) for tracking said digital control signal for a first predetermined period of time, storing said digital control signal as a digital reference signal at the end of said first period of time and comparing said digital reference signal to said digital control signal following said first predetermined period of time.

**Revendications**

1. Procédé pour déterminer la présence de sang dans le courant de dialysat d'un système d'hémodialyse au moyen d'une lumière envoyée sur le courant de dialysat, qui est périodiquement interrompue, procédé caractérisé par

— l'émission d'un pinceau lumineux dans le courant de dialysat, d'un côté de celui-ci, dans une direction rencontrant l'axe central du courant sous un angle d'incidence consigne par rapport à une droite normale à l'axe central,
— la réception de la lumière réfléchie dans un plan essentiellement commun avec le pinceau lumineux et du même côté du courant de dialysat, de sorte qu'il forme avec la normale un angle de réflexion pratiquement égal à l'angle d'incidence;
— la détermination de la valeur moyenne de courant continu du signal électrique produit par la lumière réfléchie et
— la production d'un signal d'alarme dès que la valeur moyenne de courant continu dépasse une valeur déterminée à l'avance.

2. Procédé suivant la revendication 1, caractérisé par

— la soustraction d'une tension de déplacement déterminée à l'avance de la valeur moyenne du courant continu,
— la transformation du signal réduit en un signal numérique,
— la mise en mémoire du signal numérique en tant que signal de référence numérique au bout d'un premier laps de temps déterminé à l'avance,
— la comparaison répétée du signal de référence numérique avec le signal numérique faisant suite au premier laps de temps,
— la production du signal avertisseur dès que le signal numérique varie par rapport au signal de référence numérique.

3. Dispositif de détection destine à déterminer la présence de sang dans le courant de dialysat d'un système d'hémodialyse au moyen d'une lumière envoyée sur le courant de dialysat, qui est interrompue périodiquement, selon la revendication 1, caractérisé par:

— une canalisation (19) pour le courant de dialysat, un émetter optique (18) qui est disposé sur un côté de la canalisation (19), pour envoyer un pinceau de lumière sous un angle d'incidence (Θ) déterminé à l'avance par rapport à une droite (32) normale à l'axe central (30) de la canalisation (19) dans la solution de dialysat,
— un récepteur optique (20) qui est disposé du même côté de la canalisation (19) que l'émetteur (18) et pratiquement dans le même plan sous un angle de réflexion (σ) déterminé à l'avance par rapport au plan normal pour recevoir la lumière réfléchie par la solution de dialysat, l'angle de réflexion étant pratiquement égal à l'angle d'incidence déterminé à l'avance,
— un dispositif destiné à capter la sortie du récepteur optique,
— un dispositif destiné à produire un signal de sortie en courant continu, qui réagit à la valeur moyenne en courant continu du signal de départ capté, et
— un dispositif réagissant au signal de sortie en courant continu pour émettre un signal d'alarme lorsque le signal de sortie en courant continu dépasse une valeur déterminée.

4. Dispositif suivant la revendication 3, caractérisé en ce que l'angle d'incidence (θ) déterminé à l'avance est pratiquement d'environ 55°.

5. Dispositif suivant la revendication 3, caractérisé en ce que le dispositif (42) comprend, pour la conduite intermittente d'impulsions en courant continu à travers l'émetteur optique, un oscillateur (44) et un dispositif (40) pour produire un courant constant pendant un laps de temps déterminé de chaque cycle d'oscillation.

6. Dispositif suivant la revendication 3, caractérisé en ce que le dispositif de production d'un signal de sortie en courant continu comprend un détecteur d'enveloppe des crêtes (46).

7. Dispositif suivant la revendication 3, caractérisé en ce que le dispositif destiné à émettre un signal d'alarme comprend un amplificateur différential (A4) dont une entrée est reliée à la sortie du signal en courant continu (48) et dont la seconde entrée est reliée à la sortie d'une source de référence en courant continu réglable, un dispositif (60) pour la transformation de la sortie (52) de l'amplificateur de différence (A4) en un signal de contrôle numérique et un microcalculateur (64) pour suivre le signal de contrôle numérique pendant un premier laps de temps déterminé à l'avance, pour mettre en mémoire le signal de contrôle numérique à la fin du premier laps de temps sous forme de signal de référence numérique et pour comparer le signal de référence numérique avec le signal de contrôle numérique qui suit le premier laps de temps déterminé à l'avance.

FIG. I

R1

Vcc

R2

R3

SW1

R4

0 004 599

FIG. 2

FIG. 3

F I G. 4